# EUROPEAN PATENT SPECIFICATION

(11) **EP 2 753 258 B1**
(45) Date of publication and mention of the grant of the patent: **06.11.2019**
(21) Application number: 12762671.1
(22) Date of filing: 06.09.2012
(51) Int. Cl.: A61B 18/02

(54) **CRYOPROBE**
KRYOSONDE
CRYOSONDE

(30) Priority: 08.09.2011 GB 201115504
(43) Date of publication of application: 16.07.2014
(73) Proprietor: University College Cardiff Consultants Limited, Cardiff, South Glamorgan CF24 0DE (GB)
(72) Inventor: QUANTOCK, Andrew, Bridgend South Glamorgan CF312EW (GB)
(74) Representative: Myint, Julie Marie
(86) International application number: PCT/GB2012/052183
(87) International publication number: WO 2013/034907

(56) References cited:
- US-A- 3 439 680
- US-A- 3 993 075
- Frederick Web ET AL: "Trans Am Ophthalmol Soc / Vol 106 / 2008 LIQUID NITROGEN CRYOTHERAPY FOR SURFACE EYE DISEASE (AN AOS THESIS)", Trans Am Ophthalmol Soc, 1 January 2008 (2008-01-01), XP055455990, Retrieved from the Internet: URL:https://www.ncbi.nlm.nih.gov/pmc/artic les/PMC2646430/pdf/1545-6110_v106_p301.pdf
- John S Minkowski ET AL: "Corneal Endofhelial Function and Structure Following Cryo-lnjury in the Rabbit", , 1 January 1984 (1984-01-01), XP055587426, Retrieved from the Internet: URL:https://www.google.com/url?sa=t&rct=j& q=&esrc=s&source=web&cd=2&ved=2ahUKEwjT4Pm Kp5DiAhUDMuwKHbcTAPIQFjABegQIBBAB&url=http %3A%2F%2Fiovs.arvojournals.org%2Fdata%2Fjo urnals%2Fiovs%2F933344%2F1416.pdf&usg=AOvV aw2JBywNidB-rWKQo-oDB608 [retrieved on 2019-05-10]

## Description

The invention relates to a medical device, including interchangeable parts thereof, for performing corneal cell freezing and a kit of parts including said device and/or interchangeable parts thereof.

### Introduction

The cornea is an avascular and transparent convex structure that forms a major refractive structure (around 60% of total light refraction) for light entering the eye. The cornea is made up of 5 layers; (i) the epithelium consisting non-keratinised cells, (ii) the Bowman's layer, (iii) a collagenous matrix forming the stroma, (iv) the Descemet Membrane representing the basement membrane, and (v) the corneal endothelium.

For functional vision, corneal transparency is required for clear passage of light stimuli onto the retina of the eye. Transparency is maintained by the careful control of tissue homeostasis of the multicellular layers of the cornea. Of these, the corneal endothelium forms a barrier between the stroma and aqueous humour where it acts as a metabolically-active bicarbonate pump to remove excess water from the stroma, maintaining a constant level of stromal hydration and corneal transparency.

Human corneal endothelial cells exhibit poor proliferative capacity *in vivo,* and suffer from irreversible corneal endothelium functional disorders due to disease, cell trauma and injury by ophthalmic surgery. To account for loss of cells, healing of the endothelial monolayer by sliding and enlargement of adjacent endothelial cells occurs, reducing the overall cell density of the tissue layer. Consequently, this may have an impact upon fluid regulation. Corneal endothelial disease encompasses a collection of disorders exhibiting damaged or impaired corneal endothelial cells. When such cellular dysfunction reaches a threshold limit at which the ability to maintain corneal deturgescence is lost, excess water enters the stroma with a resultant loss in transparency as a consequence of collagen fibril disruption. This eventually results in opacification of the cornea and subsequent visual impairment.

Examples of the most common sight-threatening corneal endothelial dysfunctions or disorders include bullous keratopathy and Fuchs endothelial dystrophy. Fuchs' dystrophy is often accompanied by painful bullae and eventual scarring. This is an example of a primary corneal endothelopathy, with others including congenital hereditary endothelial dystrophy and irodocorneal endothelial syndrome. So-called secondary corneal endothelialopathies can also occur as an unwanted consequence of cataract surgery (i.e. bullous keratopathy), Bourne W.D. (2003).

Corneal endothelial dysfunction often results in severe pain due to oedema and blistering, blurred vision, tearing, redness, and extreme sensitivity to light. Partial alleviation of these symptoms can be achieved through use of saline drops or therapeutic bandage contact lenses but these do not present a permanent treatment. Further palliation of these symptoms may be provided by use of anterior stromal micropuncture, an invasive technique whereby around 20 micro-needle punctures are made through the cornea into the stroma to drain excess fluid in an attempt to reduce swelling. More recently, this has been achieved through use of an Nd:YAG laser to puncture the epithelium. However, complications exist with such procedures including corneal perforation, scarring and astigmatism. Furthermore, recurrence can commonly occur.

In the cases in which irreversible corneal endothelial failure occurs, severe corneal swelling ensues with treatment often requiring surgical intervention and complete corneal transplantation. In corneal transplantation (or grafting), the damaged or diseased cornea is replaced by donated corneal tissue in its entirety (penetrating keratoplasty) or in part (lamellar keratoplasty). These techniques however are often adopted when more than just the corneal endothelium is dysfunctional or diseased. NHS data show that the number of corneal graft operations performed annually in the UK is increasing steadily, with a dramatic switch away from full-thickness penetrating grafts to more technically demanding lamellar procedures, especially endothelial keratoplasties. Each year in the US over 25,000 grafts are performed, and endothelial dysfunction is the cause of almost 60% of these. Currently, there is no alternative to keratoplasty for corneal endothelial dysfunction.

Recent techniques allow for more selective removal of the damaged cornea, with endokeratoplasty specifically targeted at removal of the corneal endothelium. One such method is Descemet's Stripping (Automated) Endothelial Keratoplasty (DSEK [or DSAEK]), in which the endothelium and Descemet membrane are specifically removed through a small peripheral corneal incision and replaced with a donor lenticule of posterior cornea tissue. Consequently, this method of surgery has improved outcome and shorter recovery times, with improved selectivity of the transplantation procedure (US2010211051). The methods are however still invasive with associated risk of infection, and donor graft failure can occur, even with development of synthetic and in *vivo* cultured corneal tissue.

A more recent alternative strategy has focussed toward removal of diseased or dysfunctional endothelial tissue, and promotion of endothelial repair and healing of the corneal tissue, thus removing the requirement for tissue transplant. Despite the fact that human corneal endothelial cells have poor proliferative capacity *in vivo,* they do retain the capacity to proliferate. For example, it has been reported that human corneal endothelial cells in organ culture proliferate in response to wounding if released from contact inhibition by EDTA in the presence of suitable growth factors. Recent research has therefore focussed on investigating methods by which enhancement of corneal healing can be achieved such that the naturally inherent poor ability of corneal cell repair can be improved. One such method is as reported by Okumura *et al* 2011 who showed that Rho associated kinase inhibitos (ROCK) promote corneal endothelial wound healing both *in vivo* and *in vitro.*

Different methods by which corneal endothelial ablation can be achieved have previously been shown. One such method is that of mechanical scraping by the surgeon through a peripheral incision, which has the obvious disadvantage of being invasive. A readily adopted non-invasive alternative method for corneal endothelial cell removal is the use of cryotherapy for transcorneal freezing, in which a fine cryoprobe freezes diseased cells. One of the advantages of this technique is that it is non-invasive i.e. the croyoprobe does not need to touch the endothelium. Nonetheless, use of the cryoprobe does ensure cell death and permeabilisation of the cornea. This non-invasive method achieves cell death by several mechanisms, such as the formation of intracellular ice which is lethal to the cell or formation of extracellular ice, causing dehydration of the cell and a subsequent increase in intracellular electrolytes and collapse of cellular membranes. The observed result is that at the margin of frozen wound sites, extensive cellular proliferation ensues wherein lost cells are replaced by new cells (Buco *et al.,* 1978; Staatz & Van Horn, 1980). Cryotherapy has long been used to eradicate corneal lesions, and it has been shown that different corneal layers exhibit different susceptibility to cell death with greatest cell death observed in the corneal endothelium (Oh *et al.,* 2010).

It has been documented that several factors affect the efficacy of cryodestruction including the cooling rate, the target tissue temperature (that to which it is cooled), the freeze-thaw cycle and number of repetitions. Moreover, *in vitro,* the use of cryoprotectants will influence cell death. Therefore the cellular layer targeted by applying the frozen probe depends on a number of parameters, which need to be controlled to produce repetitive and controlled outcome. One of the most variable parameters is the temperature to which the tissue can be cooled, and this partly depends upon application of the probe to the cell surface. Modern cryodestruction commonly involves use of a small round finger-like cryotip applicator to deliver an acute freezing stimulus to the target site. Cryotherapy is usually performed for glaucoma or retinal reattachment surgery and the cryoprobe is pressed onto the outside of the eyeball on the sclera exerting pressure inwardly, (Chapter 126, "Retinal Reattachments" in the textbook Retina (2nd Edition) Volume 3, "Surgical Retina", Glasser BM (editor), Mosby Publishers). This can result in severe wounding such that further complications can arise, such as inflammation, which have been shown to reduce the rate of endothelial regeneration (Staatz & Van Horn, 1980). Consequently, this does not aid treatment of the disorder as effective tissue regeneration is required to repair the wound tissue.

Other devices for cryotreatments of eye are described in publication US 3439680.

We have therefore developed a device to facilitate less invasive alternative treatment for corneal endothelial dysfunction that can be peformed under topical anaesthesia. Our device permits preferential controlled freezing of the corneal endothelium, without exerting damaging pressure and following a single use, which consequently shows improved tissue regeneration. Our device utilises a unique cryoprobe tip surface that permits a broad and homogenous frozen stimulus to be applied to the surface of the cornea specifically targeting diseased cells and negating the possibility of affecting subsequent tissue regeneration. The development of a device that can offer a non-invasive treatment for corneal dysfunction compared to standard procedures avoids the risk of intra-operative complications and promotes the treatment of patients in a cost-effective manner. This is of significant benefit to healthcare systems, reducing waiting times and improving patient recovery time and outcome.

### Statements of Invention

The invention is defined by the claims.

According to a first aspect of the invention there is provided a device for performing corneal freezing comprising: an elongate member having an inner inward and return flow line system through which a cryogenic fluid flows to and from an expansion chamber, respectively and, at a first end, a sealed cryoprobe tip and, at a second end, a thermal insulating handle; wherein said cryoprobe tip is fixed and comprises a concave epithelial surface contact member in thermal communication with said expansion chamber.

According to the invention, the size of said concave epithelial surface contact member is selected from one of the following options 1mm, 2mm, 3mm, 4mm, or 5mm, or 1mm plus 0.1, 0.2, 0.3, 0.4, 0.5, 0.6, 0.7, 0.8, or 0.9mm, 2mm plus 0.1, 0.2, 0.3, 0.4, 0.5, 0.6, 0.7, 0.8, or 0.9mm, 3mm plus 0.1, 0.2, 0.3, 0.4, 0.5, 0.6, 0.7, 0.8, or 0.9mm, 4mm plus 0.1, 0.2, 0.3, 0.4, 0.5, 0.6, 0.7, 0.8, or 0.9mm, 5mm plus 0.1, 0.2, 0.3, 0.4, 0.5, 0.6, 0.7, 0.8, or 0.9mm.

According to the invention, the radius of curvature of said concave epithelial surface contact member is between 7-9mm, most ideally between 7.6 and 8.6mm including all 0.1mm integers therebetween or between 6-8mm, most ideally between 6. 1mm-7.5mm including all 0.1mm integers therebetween. Thus in this way the epithelial surface contact member is made to be compatible with the radius of curvature of the cornea to facilitate contact therewith, (Aurich et al 2011).

In a further preferred embodiment of the invention, said concave epithelial surface contact member is fabricated from fine gauge stainless steel or a similar material such that either is sufficiently durable to withstand sudden temperature changes. Indeed, when the probe is used in combination with a nitrous oxide coolant it can achieve temperature changes from room temperature down to -89°C s at the tip. Moreover, said material should be thermally conductive to ensure effective cooling of the external surface of the cryoprobe tip. It will be appreciated by those skilled in the art that any suitable thermally conductive and durable material may be utilised to make the cryoprobe tip.

In yet a further preferred embodiment of the invention said device comprises a cryoprobe tip contiguous with said elongated member, alternatively said cryoprobe tip is releasably attached to said elongate member by means of a friction fit and/or a screw fix or any other suitable means known to those skilled in the art. Ideally, where a screw fix is used the tip has an internal thread and said elongated member a complimentary external thread, or vice versa. More preferably, said tip is fixed by a friction fit augmented by lock means such as a luer lock.

In a further preferred embodiment said elongate member is thermally insulated along a substantial part of its length, ideally by tubing or the use of differentially conducting materials, such that the cryoprobe tip contact surface is preferentially cooled whereby only targeted tissue and not adjacent tissue is frozen.

In a further preferred embodiment said device comprises a supply of cryogenic liquid of a finite amount and the device is manufactured from materials that are disposable. In this embodiment the device also include a pump for circulating said liquid to and from the tip and the supply. Additionally, or alternatively, said supply is removably attached to said device so that said device can be periodically refilled with said supply of liquid. Alternatively still, the device is adapted such that the inner flow line system can be connected to a coolant reservoir or supply, for delivery and retrieval of the cryogenic coolant. Ideally, the flow line system delivers a compressed cryogenic liquid.

In use cryogenic liquid is delivered to the device and, via the inward flow line, to the expansion chamber where it decompresses instantaneously by the Joules-Thomson effect, cooling down the cryoprobe tip that is in thermal communication therewith. This therefore permits the user to perform corneal freezing and so removal of corneal endothelial cells by applying the cooled contact member of the tip to the cornea, in the knowledge that a homogenous, single, non-damaging pressure stimulus is applied by the concave surface. Advantageously, this results in more rapid wound re-epithelialisation. Without wishing to be constrained by any explanation, we believe this is because a more uniform treatment engenders uniform re-epithelialisation of the corneal surface. Further, because our device avoids the application of inward pressure it also provides a more uniform and reproducible area of endothelial damage which, again promotes healing.

According to a second aspect of the invention there is provided a releasable cryoprobe tip for attaching to a device for performing corneal freezing comprising, at a first end, a releasable attachment means and, at a second end, a concave epithelial surface contact member.

In a preferred embodiment of the invention said cryoprobe tip also comprises the whole or a part of an expansion chamber which is in thermal communication with said tip. Ideally, said tip also comprises a flow line adapted to be connected to a flow line system for cryogenic fluid provided in said device for performing corneal freezing.

According to a further aspect of the invention there is provided a kit of parts for use in a device for performing corneal freezing comprising a plurality of releasable cryoprobe tips having, at a first end, a releasable attachment means and, at a second end, a concave epithelial surface contact member.

In this preferred embodiment of the invention, ideally, said tips are of different sizes and so of different diameters and/or angles of curvature as herein described. The range of probe sizes will allow the destruction of different sized areas of diseased endothelium depending upon the disease stage and extent of endothelial damage.

It will further be apparent to those skilled in the art that the device is used for medical purposes.

Most preferably, said device is used to remove corneal endothelial cells in the treatment of corneal endothelial disorders such as, but not limited to, bullous keratopathy and Fuchs endothelial dystrophy.

Any of the aforementioned aspects of the invention may, in preferred embodiments, include or be characterised by any of the aforementioned features pertaining to the device or the member or the tips. Thus, preferred features of each aspect of the invention may be as described in connection with any of the other aspects.

In the claims which follow and in the preceding description of the invention, except where the context requires otherwise due to express language or necessary implication, the word "comprises", or variations such as "comprised" or "comprising" is used in an inclusive sense i.e. to specify the presence of the stated features but not to preclude the presence or addition of further features in various embodiments of the invention.

Other features of the present invention will become apparent from the following examples.

Moreover, unless stated otherwise, any feature disclosed herein may be replaced by an alternative feature serving the same or a similar purpose.

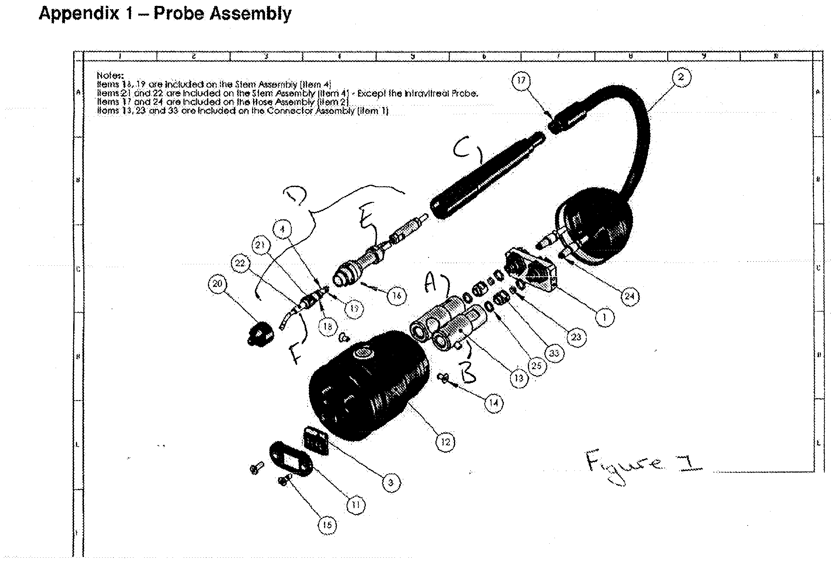

The invention will now be described by way of example only with reference to the following figures, wherein:-
Figure 1 shows an exploded perspective view of a device for performing corneal freezing;
Figure 2 shows a side elevation view of the tip of the device shown in figure 1;
Figure 3 shows a posterior elevation view of the tip of the device shown in figure 1; and
Figure 4 shows a side elevation view of the end of the device shown in figures 2 & 3;
Referring now to the figures and, in particular figure 1, there is shown a device typically used to perform corneal freezing. The device shown in figure 1 is adapted for secure attachment to a supply of cryogenic fluid such as a supply of liquid coolant in a clinical environment. Usually, the liquid coolant is a compressed supply of coolant such as nitrous oxide. However, those skilled in the art will appreciate that any coolant may be used.

The device comprises an inner flow system for transporting said coolant to and from both a tip supplied on the end of the device and the said supply of coolant. Accordingly, said flow system has an inward and outward, or return, flow conduit designating by A and B in figure 1. Although not shown, conduits A & B travel along the entire length of the device and at the tip, or end 4, of the device communicate with an expansion chamber, also not shown.

As is illustrated in figure 1, the device includes a number of conventional gaskets, washers and bolts for securely and hermetically connecting the device to a coolant supply. This is to ensure coolant cannot escape or spill from the system. Those skilled in the art will appreciate that the invention is not to be limited to the connection arrangement shown in figure 1, rather this is shown for illustrative purposes only. Any other connection arrangement that securely attaches the device to a coolant supply and known, or deducible, by those skilled in the art may be use.

In alternative embodiments of the invention, the device may be supplied as a stand-alone embodiment in which a finite amount of coolant fluid is supplied in a reservoir in fluid communication with said flow system. Further, in this arrangement, the reservoir may be releasably attached to said device using conventional means so that, periodically, said supply of coolant can be replenished. Additionally, or alternatively, this embodiment may be provided as a disposable device and so may be made from materials with this in mind.

Downstream of the connection arrangement there is provided a handle C which is made from thermally insulating material so that when the device is in use the temperature of the handle stays relatively constant, thus protecting a user from the effects of the temperature changes experienced elsewhere in said device.

Downstream of handle C there is provided a releasable tip D. Tip D is releasably connected to handle C by a conventional friction engagement mechanism and also, ideally, a screw fit arrangement. In the embodiment shown, a connector E has at a first end a friction engagement arrangement and at a second end a screw engagement arrangement. This is to ensure that the releasable tip is securely fastened to the device when in use. Accordingly, locking mechanisms may also be employed. Further, other arrangements, known to those skilled in the art may be employed in the working of the invention.

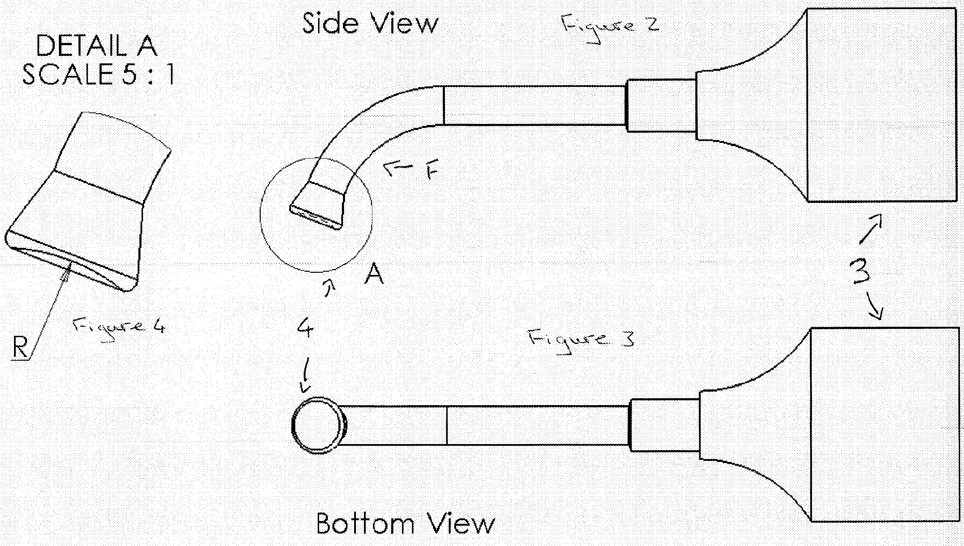

In figures 2 and 3 there is shown a side and posterior elevation view, respectively, of the tip of the device shown in figure 1. At a first end 3 there is provided an inner screw thread, not shown, for engaging with a compatible thread provided on handle C. At a second end 4 there is provided an epithelial surface contact member F. The tip of member F is typically of circular cross-section, although elliptical sections can be used in the working of the invention, and is concave. This concavity is best seen by reference to figure 4. Ideally, the member is 1, 2, 3, 4 or 5mm diameter and more ideally still the member is 1, 2, 3, 4 or 5mm diameter plus or minus 0.1, 0.2, 0.3, 0.4, 0.5, 0.6, 0.7, 0.8, or 0.9mm. Thus the size of the tip of member F may be between 1-5mm including every 0.1mm integer increase or decrease in size. The radius of curvature of the tip of member F is between 7-9mm, most ideally between 7.6 and 8.6mm including all 0.1mm integers therebetween or between 6-8mm, most ideally between 6. 1mm-7.5mm including all 0.1mm integers therebetween. Thus in this way the epithelial surface contact member is made to be compatible with the radius of curvature of the cornea to facilitate contact therewith.

Given member F can be releasably attached to handle C, a kit of parts may be provided comprising the device shown plus a number of different members F, ideally the different members F have different tip sizes or diameters and/or different radii of curvature so that, in use, a clinicians can select the tip to be used having regard to the nature or scale of the treatment to be performed.

In use, a clinician selects an appropriate tip having regard to the nature of the treatment to be performed and, if not attached to the device, attaches it to the device using connector E. Fluid coolant is then made to flow through the device by activating an appropriate pump or by connecting the device to a supply of flowing coolant. Once the clinician has ensured coolant is flowing through the device the device can be applied to corneal tissue for the purpose of performing a treatment. Using a device having the features described herein, we have discovered that the nature of the cooling is such that the remaining tissue heals advantageously quickly. Without wishing to be constrained by any explanation, we consider this is due to the fact that a more uniform treatment engenders uniform re-epithelialisation of the corneal surface. Further, because our device avoids the application of inward pressure it also provides a more uniform and reproducible area of endothelial damage which, again promotes healing.

The invention thus provides a superior device for performing corneal treatment.

### References:

Bourne W.D. (2003) Biology of the corneal endothelium in health and disease. Eye 17; 912-18).
Okumura et al (2011) Enhancement of corneal endothelium wound healing by Rho-associated kinase (ROCK) inhibitor eye drops. Br. J. Opthalmol, 95: 1006-1009.
Buco P, Van Horn DL, Schutten WH, Cohen K. (1978) Effects of transcorneal freezing on protein content of aqueous humor and intraocular temperature in rabbit and cat. Invest Ophthalmol Vis Sci. 17(12):1199-1202.
Staatz WD, Van Horn DL. (1980) The effects of aging and inflammation on corneal endothelial wound healing in rabbits. Invest Ophthalmol Vis Sci. 19(8):983-6.
Oh JY, Lee HJ, Khwarg SI, Wee WR. (2010) Corneal cell viability and structure after transcorneal freezing-thawing in the human cornea. Clin Ophthalmol. 25(4):477-80.
Aurich H, Pham DT, Wirbelauer C. 2011 Biometric evaluation of keratoconic eyes with slit lamp-adapted optical coherence tomography. Cornea. Jan;30(1):56-9).

## Claims

1. A device for performing corneal freezing comprising: an elongate member having an inner inward and return flow line system through which a cryogenic fluid flows to and from an expansion chamber, respectively and, at a first end, a sealed cryoprobe tip and, at a second end, a thermal insulating handle; wherein said cryoprobe tip is fixed and comprises a concave epithelial surface contact member in thermal communication with said expansion chamber, the device **characterised in that** the concave epithelial surface contact member has a radius of curvature between 7-9mm and a diameter selected from one of the following options 1 mm, 2mm, 3mm, 4mm, or 5mm, or 1 mm plus 0.1, 0.2, 0.3, 0.4, 0.5, 0.6, 0.7, 0.8, or 0.9mm, 2mm plus 0.1, 0.2, 0.3, 0.4, 0.5, 0.6, 0.7, 0.8, or 0.9mm, 3mm plus 0.1, 0.2, 0.3, 0.4, 0.5, 0.6, 0.7, 0.8, or 0.9mm, 4mm plus 0.1, 0.2, 0.3, 0.4, 0.5, 0.6, 0.7, 0.8, or 0.9mm, 5mm plus 0.1, 0.2, 0.3, 0.4, 0.5, 0.6, 0.7, 0.8, or 0.9mm.

2. The device according to claim 1 wherein the radius of curvature of said concave epithelial surface contact member is selected from one of the following options, 7.6-8.6mm including all 0.1mm integers therebetween, 7-8mm, 7.1 mm-7.5mm including all 0.1mm integers therebetween.

3. The device according to any preceding claim wherein said concave epithelial surface contact member is fabricated from fine gauge stainless steel.

4. The device according to any preceding claim wherein said device comprises a cryoprobe tip contiguous with said elongated member.

5. The device according to claims 1-4 wherein said cryoprobe tip is releasably attached to said elongate member.

6. The device according to any preceding claim wherein said elongate member is thermally insulated along a substantial part of its length such that the cryoprobe tip contact surface is preferentially cooled.

7. The device according to any preceding claim wherein said device comprises a supply of cryogenic liquid of a finite amount and said device further includes a pump for circulating said liquid to and from the tip and the supply.

8. The device according to claim 7 wherein said supply is removably attached to said device so that said device can be periodically refilled with said supply of liquid.

9. The device according to claims 7 or 8 wherein the device is manufactured from materials that are disposable.

10. The device according to claims 1- 6 wherein the device is adapted such that the inner flow line system can be connected to a coolant reservoir or supply, for delivery and retrieval of the cryogenic coolant.

11. The device according to claim 10 wherein said coolant is a compressed cryogenic liquid.

12. A releasable cryoprobe tip for attaching to a device for performing corneal freezing comprising, at a first end, a releasable attachment means and, at a second end, a concave epithelial surface contact member, **characterised in that** the concave epithelial surface contact member has a radius of curvature between 7-9mm and a diameter selected from one of the following options 1mm, 2mm, 3mm, 4mm, or 5mm, or 1mm plus 0.1, 0.2, 0.3, 0.4, 0.5, 0.6, 0.7, 0.8, or 0.9mm, 2mm plus 0.1, 0.2, 0.3, 0.4, 0.5, 0.6, 0.7, 0.8, or 0.9mm, 3mm plus 0.1, 0.2, 0.3, 0.4, 0.5, 0.6, 0.7, 0.8, or 0.9mm, 4mm plus 0.1, 0.2, 0.3, 0.4, 0.5, 0.6, 0.7, 0.8, or 0.9mm, 5mm plus 0.1, 0.2, 0.3, 0.4, 0.5, 0.6, 0.7, 0.8, or 0.9mm

13. The releasable cryoprobe tip according to claim 12 wherein said cryoprobe tip also comprises the whole or a part of an expansion chamber which is in thermal communication with said tip.

14. The releasable cryoprobe tip according to claim 13 wherein said tip also comprises a flow line adapted to be connected to a flow line system for cryogenic fluid provided in said device for performing corneal freezing.

15. A kit of parts for use in a device for performing corneal freezing comprising a plurality of releasable cryoprobe tips having, at a first end, a releasable attachment means and, at a second end, a concave epithelial surface contact member, **characterised in that** the concave epithelial surface contact member has a radius of curvature between 7-9mm and a diameter selected from one of the following options 1mm, 2mm, 3mm, 4mm, or 5mm, or 1mm plus 0.1, 0.2, 0.3, 0.4, 0.5, 0.6, 0.7, 0.8, or 0.9mm, 2mm plus 0.1, 0.2, 0.3, 0.4, 0.5, 0.6, 0.7, 0.8, or 0.9mm, 3mm plus 0.1, 0.2, 0.3, 0.4, 0.5, 0.6, 0.7, 0.8, or 0.9mm, 4mm plus 0.1, 0.2, 0.3, 0.4, 0.5, 0.6, 0.7, 0.8, or 0.9mm, 5mm plus 0.1, 0.2, 0.3, 0.4, 0.5, 0.6, 0.7, 0.8, or 0.9mm

16. The kit of parts according to claim 15 wherein said tips are of different sizes and so of different diameters and/or angles of curvature.

## Patentansprüche

1. Vorrichtung für das Durchführen einer Hornhautvereisung, Folgendes umfassend: ein längliches Element mit einem internen Zufluss- und Rückflussleitungssystem, durch das eine kryogene Flüssigkeit zu und von einer Expansionskammer fließt und das am ersten Ende eine versiegelte Kryosondenspitze sowie am zweiten Ende einen wärmeisolierenden Handgriff aufweist; wobei die Kryosondenspitze fixiert ist und ein konkaves epitheliales Oberflächenkontaktelement umfasst, das in thermischer Kommunikation mit der Expansionskammer steht, wobei die Vorrichtung **dadurch gekennzeichnet ist, dass** das konkave, epitheliale Oberflächenkontaktelement einen Krümmungsradius von zwischen 7-9 mm und einen Durchmesser aufweist, der aus den folgenden Optionen ausgewählt wird: 1 mm, 2 mm, 3 mm, 4 mm oder 5mm, oder 1 mm plus 0,1, 0,2, 0,3, 0,4, 0,5, 0,6, 0,7, 0,8 oder 0,9 mm, 2 mm plus 0,1, 0,2, 0,3, 0,4, 0,5, 0,6, 0,7, 0,8 oder 0,9 mm, 3 mm plus 0,1, 0,2, 0,3, 0,4, 0,5, 0,6, 0,7, 0,8 oder 0,9 mm, 4 mm plus 0,1, 0,2, 0,3, 0,4, 0,5, 0,6, 0,7, 0,8 oder 0,9 mm, 5 mm plus 0,1, 0,2, 0,3, 0,4, 0,5, 0,6, 0,7, 0,8 oder 0,9 mm.

2. Vorrichtung nach Anspruch 1, wobei der Krümmungsradius des konkaven epithelialen Oberflächenelements aus einer der folgenden Optionen ausgewählt wird: 7,6-8,6 mm, einschließlich aller dazwischenliegenden 0,1 mm Ganzzahlen, 7-8 mm, 7,1 mm - 7,5 mm, einschließlich aller dazwischenliegenden 0,1 mm Ganzzahlen.

3. Vorrichtung nach einem der vorhergehenden Ansprüche, wobei das besagte konkave epitheliale Oberflächenkontaktelement aus feinem Edelstahl hergestellt ist.

4. Vorrichtung nach einem der vorhergehenden Ansprüche, wobei die Vorrichtung eine Kryosondenspitze umfasst, die mit dem länglichen Element zusammenhängend ist.

5. Vorrichtung nach einem der Ansprüche 1-4, wobei die Kryosondenspitze lösbar an dem länglichen Element angebracht ist.

6. Vorrichtung nach einem der vorhergehenden Ansprüche, wobei das längliche Element entlang eines wesentlichen Teils seiner Länge wärmeisoliert ist, sodass die Kontaktoberfläche der Kryosondenspitze vorzugsweise gekühlt ist.

7. Vorrichtung nach einem der vorhergehenden Ansprüche, wobei die Vorrichtung eine Versorgung mit kryogener Flüssigkeit in einer begrenzenten Menge umfasst und die Vorrichtung ferner eine Pumpe für die Zirkulation der Flüssigkeit zur und von der Spitze und der Versorgung beinhaltet.

8. Vorrichtung nach Anspruch 7, wobei die besagte Versorgung lösbar an der Vorrichtung angebracht ist, sodass die Vorrichtung regelmäßig mit der Versorgung an Flüssigkeit wieder aufgefüllt werden kann.

9. Vorrichtung nach Anspruch 7 oder 8, wobei die Vorrichtung aus wegwerfbaren Werkstoffen gefertigt ist.

10. Vorrichtung nach Anspruch 1 bis 6, wobei die Vorrichtung so angepasst ist, dass das innere Flussleitungssystem an einen Kühlmitteltank oder eine Kühlmittelversorgung angeschlossen werden kann, für die Bereitstellung und die Rückführung des kryogenen Kühlmittels.

11. Vorrichtung nach Anspruch 10, wobei das Kühlmittel eine komprimierte kryogene Flüssigkeit ist.

12. Lösbare Kryosondenspitze für das Anbringen auf einer Vorrichtung für das Durchführen einer Hornhautvereisung, Folgendes umfassend: an einem ersten Ende ein lösbares Befestigungsmittel und an einem zweiten Ende ein konkaves epitheliales Oberflächenkontaktelement, **dadurch gekennzeichnet, dass** das konkave, epitheliale Oberflächenkontaktelement einen Krümmungsradius zwischen 7-9 mm sowie einen Durchmesser aufweist, der aus einer der folgenden Optionen ausgewählt ist: 1 mm, 2 mm, 3 mm, 4 mm oder 5 mm, oder 1 mm plus 0,1, 0,2, 0,3, 0,4, 0,5, 0,6, 0,7, 0,8 oder 0,9 mm, 2 mm plus 0,1, 0,2, 0,3, 0,4, 0,5, 0,6, 0,7, 0,8 oder 0,9 mm, 3 mm plus 0,1, 0,2, 0,3, 0,4, 0,5, 0,6, 0,7, 0,8 oder 0,9 mm, 4 mm plus 0,1, 0,2, 0,3, 0,4, 0,5, 0,6, 0,7, 0,8 oder 0,9 mm, 5 mm plus 0,1, 0,2, 0,3, 0,4, 0,5, 0,6, 0,7, 0,8 oder 0,9 mm

13. Lösbare Kryosondenspitze nach Anspruch 12, wobei die besagte Kryosondenspitze auch die gesamte oder einen Teil der Expansionskammer umfasst, die in thermischer Kommunikation mit der Spitze steht.

14. Lösbare Kryosondenspitze nach Anspruch 13, wobei die Spitze auch eine Flussleitung umfasst, die so angepasst ist, dass sie an ein Flussleitungssystem für die kryogene Flüssigkeit, die in der Vorrichtung für das Durchführen der Hornhautvereisung bereitgestellt wird, angeschlossen werden kann.

15. Satz an Teilen für die Verwendung in einer Vorrichtung für die Durchführung der Hornhautvereisung, Folgendes umfassend: eine Vielzahl an lösbaren Kryosondenspitzen, die am ersten Ende ein lösbares Befestigungsmittel und am zweiten Ende ein konkaves epitheliales Oberflächenkontaktelement aufweisen, **dadurch gekennzeichnet, dass** das epitheliale Oberflächenkontaktelement einen Krümmungsradius zwischen 7-9 mm und einen Durchmesser aufweist, der aus einer der folgenden Optionen ausgewählt wird: 1 mm, 2 mm, 3 mm, 4 mm oder 5 mm, oder 1 mm plus 0,1, 0,2, 0,3, 0,4, 0,5, 0,6, 0,7, 0,8 oder 0,9 mm, 2 mm plus 0,1, 0,2, 0,3, 0,4, 0,5, 0,6, 0,7, 0,8 oder 0,9 mm, 3 mm plus 0,1, 0,2, 0,3, 0,4, 0,5, 0,6, 0,7, 0,8 oder 0,9 mm, 4 mm plus 0,1, 0,2, 0,3, 0,4, 0,5, 0,6, 0,7, 0,8 oder 0,9 mm, 5 mm plus 0,1, 0,2, 0,3, 0,4, 0,5, 0,6, 0,7, 0,8 oder 0,9 mm

16. Satz an Teilen nach Anspruch 15, wobei die Spitzen unterschiedliche Größen und daher auch unterschiedliche Durchmesser und/oder Krümmungswinkel aufweisen.

## Revendications

1. Dispositif permettant d'effectuer une congélation cornéenne comprenant : un élément allongé ayant un système de conduite d'écoulement interne vers l'intérieur et de retour à travers lequel un fluide cryogénique s'écoule vers et depuis une chambre de dilatation, respectivement, et au niveau d'une première extrémité, un embout de cryosonde scellé, et au niveau d'une seconde extrémité, une poignée thermiquement isolante ; dans lequel ledit embout de cryosonde est fixe et comprend un élément de contact de surface épithéliale concave en communication thermique avec ladite chambre de dilatation, le dispositif étant **caractérisé en ce que** l'élément de contact de surface épithéliale concave a un rayon de courbure compris entre 7 et 9 mm et un diamètre choisi parmi l'une des options suivantes : 1 mm, 2 mm, 3 mm, 4 mm, ou 5 mm ou 1 mm plus 0,1, 0,2, 0,3, 0,4, 0,5, 0,6, 0,7, 0,8 ou 0,9 mm, 2 mm plus 0,1, 0,2, 0,3, 0,4, 0,5, 0,6, 0,7, 0,8 ou 0,9 mm, 3 mm plus 0,1, 0,2, 0,3, 0,4, 0,5, 0,6, 0,7, 0,8 ou 0,9 mm, 4 mm plus 0,1, 0,2, 0,3, 0,4, 0,5, 0,6, 0,7, 0,8 ou 0,9 mm, 5 mm plus 0,1, 0,2, 0,3, 0,4, 0,5, 0,6, 0,7, 0,8 ou 0,9 mm.

2. Dispositif selon la revendication 1, dans lequel le rayon de courbure dudit élément de contact de surface épithéliale concave est choisi parmi l'une des options suivantes, 7,6 à 8,6 mm, y compris tous les entiers 0,1 mm entre eux, 7 à 8 mm, 7,1 mm à 7,5 mm, y compris tous les entiers de 0,1 mm entre eux.

3. Dispositif selon une quelconque revendication précédente, dans lequel ledit élément de contact de surface épithéliale concave est fabriqué à partir d'acier inoxydable de calibre fin.

4. Dispositif selon une quelconque revendication précédente, dans lequel ledit dispositif comprend un embout de cryosonde contiguë audit élément allongé.

5. Dispositif selon les revendications 1 à 4, dans lequel ledit embout de cryosonde est fixé de manière amovible audit élément allongé.

6. Dispositif selon une quelconque revendication précédente, dans lequel ledit élément allongé est isolé thermiquement sur une partie substantiel de sa longueur, de sorte que la surface de contact de l'embout de cryosonde est de préférence refroidie.

7. Dispositif selon une quelconque revendication, dans lequel ledit dispositif comprend une alimentation en liquide cryogénique d'une quantité finie et ledit dispositif comprend en outre une pompe pour faire circuler ledit liquide vers et depuis l'embout et l'alimentation.

8. Dispositif selon la revendication 7, dans lequel ladite alimentation est fixée de manière amovible audit dispositif de sorte que ledit dispositif peut être rempli périodiquement avec ladite alimentation de liquide.

9. Dispositif selon les revendications 7 ou 8, dans lequel le dispositif est fabriqué à partir de matériaux détachables.

10. Dispositif selon les revendications 1 à 6, dans lequel le dispositif est conçu de sorte que le système de conduite d'écoulement interne peut être relié à un réservoir ou à une alimentation de réfrigérant, pour la distribution et la récupération du réfrigérant cryogénique.

11. Dispositif selon la revendication 10, dans lequel ledit réfrigérant est un liquide cryogénique comprimé.

12. Embout de cryosonde amovible destiné à être fixé à un dispositif permettant d'effectuer une congélation cornéenne comprenant, au niveau d'une première extrémité, un moyen de fixation amovible, et au niveau d'une seconde extrémité, un élément de contact de surface épithéliale concave, **caractérisé en ce que** l'élément de contact de surface épithéliale concave a un rayon de courbure compris entre 7 et 9 mm et un diamètre choisi parmi l'une des options suivantes : 1 mm, 2 mm, 3 mm, 4 mm ou 5 mm ou 1 mm plus 0,1, 0,2, 0,3, 0,4, 0,5, 0,6, 0,7, 0,8 ou 0,9 mm, 2 mm plus 0,1, 0,2, 0,3, 0,4, 0,5, 0,6, 0,7, 0,8 ou 0,9 mm, 3 mm plus 0,1, 0,2, 0,3, 0,4, 0,5, 0,6, 0,7, 0,8 ou 0,9 mm, 4 mm plus 0,1, 0,2, 0,3, 0,4, 0,5, 0,6, 0,7, 0,8 ou 0,9 mm, 5 mm plus 0,1, 0,2, 0,3, 0,4, 0,5, 0,6, 0,7, 0,8 ou 0,9 mm

13. Embout de cryosonde amovible selon la revendication 12, dans lequel ledit embout de cryosonde comprend également la totalité ou une partie d'une chambre de dilatation qui est en communication thermique avec ledit embout.

14. Embout de cryosonde amovible selon la revendication 13, dans lequel ledit embout comprend également une conduite d'écoulement conçue pour être reliée à un système de conduite d'écoulement pour un fluide cryogénique prévu dans ledit dispositif permettant d'effectuer une congélation cornéenne.

15. Kit de pièces à utiliser dans un dispositif permettant d'effectuer une congélation cornéenne comprenant une pluralité d'embouts de cryosonde amovibles ayant, au niveau d'une première extrémité, un moyen de fixation amovible et, au niveau d'une seconde extrémité, un élément de contact de surface épithéliale concave, **caractérisé en ce que** l'élément de contact de surface épithéliale concave a un rayon de courbure compris entre 7 et 9 mm et un diamètre choisi parmi l'une des options suivantes : 1 mm, 2 mm, 3 mm, 4 mm ou 5 mm, ou 1 mm plus 0,1, 0,2, 0,3, 0,4, 0,5, 0,6, 0,7, 0,8 ou 0,9 mm, 2 mm plus 0,1, 0,2, 0,3, 0,4, 0,5, 0,6, 0,7, 0,8 ou 0,9 mm, 3 mm plus 0,1, 0,2, 0,3, 0,4, 0,5, 0,6, 0,7, 0,8 ou 0,9 mm, 4 mm plus 0,1, 0,2, 0,3, 0,4, 0,5, 0,6, 0,7, 0,8 ou 0,9 mm, 5 mm plus 0,1, 0,2, 0,3, 0,4, 0,5, 0,6, 0,7, 0,8 ou 0,9 mm

16. Kit de pièces selon la revendication 15, dans lequel lesdits embouts sont de tailles et donc de diamètres et/ou d'angles de courbure différents.
